# EUROPÄISCHE PATENTSCHRIFT

(11) **EP 0 650 359 B1**
(45) Veröffentlichungstag und Bekanntmachung des Hinweises auf die Patenterteilung: **28.04.1999**
(21) Anmeldenummer: 93915819.2
(22) Anmeldetag: 07.07.1993
(51) Int. Cl.: A61K 31/565, A61K 9/70

(54) **TRANSDERMALES THERAPEUTISCHES SYSTEM MIT DEM WIRKSTOFF 17 -ESTRADIOL (WASSERFREI)**
TRANSDERMAL THERAPEUTIC SYSTEM CONTAINING THE ACTIVE SUBSTANCE 17- -OESTRADIOL (ANHYDROUS)
SYSTEME THERAPEUTIQUE TRANSCUTANE LIBERANT DE L'ESTRADIOL 17- -(ANHYDRE)

(30) Priorität: 16.07.1992 DE 4223360
(43) Veröffentlichungstag der Anmeldung: 03.05.1995
(73) Patentinhaber: LTS LOHMANN Therapie-Systeme GmbH, 56567 Neuwied (DE)
(72) Erfinder: HORSTMANN, Michael, D-56564 Neuwied (DE); KURSAWE, Marion, D-56626 Andernach (DE); DZEKAN, Horst, D-56271 Kleinmaischeid (DE)
(74) Vertreter: Flaccus, Rolf-Dieter, Dr.
(86) Internationale Anmeldenummer: EP9301754
(87) Internationale Veröffentlichungsnummer: WO9402151

(56) Entgegenhaltungen:
- EP-A- 0 304 227
- EP-A- 0 337 358
- WO-A-90/10425
- US-A- 3 854 480

## Beschreibung

Die Erfindung betrifft ein transdermales therapeutisches System zur Abgabe von 17-β-Estradiol und gegebenenfalls werterer Wirkstoffe über die Haut an den menschlichen Körper.

Transdermale Therapeutische Systeme (TTS) sind in der arzneilichen Therapie einer Reihe von Erkrankungen bereits im Markt eingeführt. Auch TTS mit dem Wirkstoff 17-β-Estradiol sind inzwischen als Therapeutikum für Wechseljahrsbeschwerden, neuerdings auch gegen Osteoporose im Handel und bewähren sich erfolgreich in der Therapie.

Ein Nachteil der dem Stand der Technik entsprechenden Systeme ist die nicht ausreichende Permeationsfähigkeit des Wirkstoffes durch die Haut, die auch durch zahlreiche galenische Maßnahmen des TTS-Designs (Einsatz mehrschichtiger Systeme, Verwendung von Steuermembranen, Variation der Wirkstoffkonzentration, Modifikation der Grundpolymeren etc.) nicht über eine gewisse Grenze, den sogenannten "Sättigungsfluß", hinaus steigerbar ist. Diese Feststellung, daß der transdermale Fluß eines Wirkstoffes aus der festen, fein verteilten Phase heraus auch bei Einsatz stärker lösender Vehikel prinzipiell nicht weiter steigerbar ist, findet sich bereits in den auch heute noch wegweisenden Arbeiten von Higuchi (z.B. T. Higuchi: Physical Chemical Analysis of percutaneous absorption process from creams and ointments, J. Soc. Cosmetic Chem. 11,S. 85-97 (1960).

Die in EP 0 421 454 beschriebenen Systeme enthalten 17-β-Estradiol in einem Acrylatpolymer unter Zusatz von "Kristallisationshemmern" und klebrigmachenden Harzen. Quellstoffe sind zum Schutz vor vorzeitigem Klebkraftverlust enthalten.
Allerdings gibt es für viele Wirkstoffe die Möglichkeit, dem TTS bei der Herstellung sogenannte Enhancer (Penetrationsverstärker) zuzusetzen. Es handelt sich hier um in der Regel flüssige Zusatzstoffe, die die Resorptionseigenschaften der menschlichen Haut verbessern und damit die Aufnahme des Wirkstoffes aus einer genügend kleinen TTS-Fläche heraus ermöglichen. Speziell leicht flüchtige Enhancer wie das zum Beispiel für den Wirkstoff 17-β-Estradiol verwendete Ethanol bergen Probleme der allzu starken Erweichung der Klebschichten von TTS und machen vor allem weitere, raumfordernde Kompartimente im System notwendig, die das TTS unakzeptabel dick werden lassen. Schließlich birgt jeder weitere nichtpolymere Zusatzstoff auch die Gefahr von Unverträglichkeiten auf der Haut, unter Umständen auch von Sensibilisierungen. Unter Zusatz bestimmter weniger flüchtiger, meist aber auch weniger aktiver Enhancer (z.B. Glycerinester, cyclische Amide, Eucalyptol) ist zwar die Herstellung von Matrixsystemen möglich, die den Wirkstoff und die resorptionsverstärkende Komponente in einer oder mehreren monolithischen Schichten enthalten.

US 4,863,738 stellt ein Beispiel von vielen dar, in denen die Applikation von Wirkstoffen, z.B. 17-β-Estradiol, zusammen mit einem bestimmten Enhancer (hier Glycerinmonooleat) in einer beliebigen TTS-Matrix in beliebiger Konzentration beansprucht wird.
Leider ist nach dem Stand der Technik auch mit solchen TTS keine befriedigende Therapie möglich, da entweder die ausgewählten Enhancer zu schlecht hautverträglich sind oder die Systeme wegen des noch immer zu geringen Flusses durch die Haut unakzeptabel große Flächen aufweisen.

Eine andere (theoretische) Möglichkeit, den Wirkstofffluß durch die Haut zu steigern, besteht darin, mehr Wirkstoff im TTS molekulardispers aufzulösen, als der Sättigungslöslichkeit entspricht. Mit dem Ausmaß der Übersättigung solcher Systeme steigt in gleichem Maße auch die Permeationsgeschwindigkeit durch die Haut an. Da solche physikalischen Zustände jedoch thermodynamisch instabil sind, sind solche Arzneiformen nicht lagerfähig, es findet innerhalb von Monaten und spätestens Jahren eine spontane, unvorhersehbare Ausfällung von Wirkstoffteilchen statt, so daß die Flußrate durch die Haut nach und nach auf Sättigungsflußniveau abfältt und damit ein großer Teil der initial vorhandenen therapeutischen Aktivität verloren geht.

Aufgabe der Erfindung ist die Bereitstellung eines transdermalen therapeutischen Systems mit dem Wirkstoff 17-β-Estradiol, welches höhere Wirkstoffflüsse auf der Haut aufweist als dem Stand der Technik entsprechende Systeme mit dem gleichen Wirkstoff und das während der Lagerzeit (Verwendbarkeits- oder Aufbrauchsfrist) vor Aktivitätsverlusten durch Rekristallisation (Wirkstoffausfällung) geschützt ist.Weiterhin betrifft die Aufgabe ein für dessen Herstellung geeignetes Verfahren

Diese Aufgabe wird erfindungsgemäß bei einem transdermalen therapeutischen System dadurch gelöst, daß mindestens eine der Matrixschichten und/oder die Klebschicht wasserfreies 17-β-Estradiol in kristalliner Form enthält. Hierfür geeignete Herstellungsverfahren sind entsprechend den Merkmalen der Ansprüche 8 bis 10 vorgesehen.

17-β-Estradiol liegt bei Raumtemperatur und gewöhnlicher relativer Luftfeuchtigkeit (20 bis 60 % relativer Feuchte) nicht in einer der beiden bekannten kristallwasserfreien Modifikationen (I und II) vor, sondern als Semihydrat (Busetti u. Hospital, Acta Cryst. 1972, B28, 560). Wegen der schichtförmigen, über Wasserstoffbrücken stabilisierten Struktur und der Diffusionsdichtigkeit des Kristallverbundes läßt sich das Hydrat kurzzeitig unzersetzt bis zu Temperaturen von ca. 170°C erhitzen (Kuhnert-Brandstätter u. Winkler (1976) Scientia Pharmaceutica 44 (3), 177-190). Durch Mikronisieren kann 17-β-Estradiol-semihydrat jedoch auf dem Wege der Kristalloberflächenvergrößerung bereits bei ca. 120 °C quantitativ in die wasserfreie Form umgewandelt werden. Nach unseren eigenen Beobachtungen findet bei langsamem Erwärmen (0,2 bis 1 K/min) und besonders feiner Ware die Umwandlung sogar bereits bei etwa 90 °C statt.

Andererseits weist 17-β-Estradiol mit geringer werdendem Wasserdampfpartialdruck höhere Löslichkeiten in einigen Polymeren, speziell auch Polyacrylaten auf (Beispiel 1). Da mit höherer Konzentration unter sonst gleichen Bedingungen der Diffusionsfluß durch die Haut nach dem Fickschen Gesetz zunimmt, ist eine solche Konzentrationssteigerung bei transdermalen therapeutischen Systemen sehr erwünscht. Leider reicht jedoch bereits das mit dem 17-β-Estradiol-semihydrot eingebrachte Wasser aus, um nach und nach ein erneutes Auskristallisieren als 17-β-Estradiol-semihydrat aus der Lösung auszulösen. (Kuhnert-Brandstätter u. Winkler (1976) Scientia Pharmaceutica 44 (3), 177-190). Nach dem Auskristallisieren fällt mit der geringer werdenden Konzentration leider auch die Flußrate aus dem System an die Haut stark ab. Erfindungsgemäß wird daher durch sorgfältige Trocknung der schichtförmigen Bestandteile des transdermalen therapeutischen Systems sowohl eine wasserarme Matrix hergestellt, als auch z.B. durch Umwandlung aus Estradiol-Semihydrat eine thermodynamisch stabilisierende Phase aus wasserfreiem 17-β-Estradiol eingebracht oder erzeugt. Da diese Form bei Raumtemperatur die energetisch instabilere ist, weist sie eine deutlich höhere Löslichkeit auf als das Semihydrat - es entsteht also eine gesättigte Lösung mit Bodenkörper bei gegenüber luftfeucht hergestellten Systemen deutlich erhöhter freier 17-β-Estradiolkonzentration.
Selbstverständlich sind zur Stabilisierung dieser Eigenschaften absolut trockene Lagerbedingungen notwendig. Diese lassen sich durch gas- und feuchtigkeitsdichte Packmittel oder aber durch Einlegen von einem oder mehreren feuchtigkeitsabsorbierenden Körpern in das Packmittel gewährleisten.

Die vorstehend beschriebene erfindungsgemäße Verwendung von wasserfreiem Estradiol in kristalliner Form kann auf unterschiedliche Weise in einem TTS realisiert werden: Die einfachste Form ist ein einschichtiges Matrixsystem, dessen Matrix von ihrer Funktion her zugleich haftklebend ist und damit die Klebschicht überflüssig macht. Durch die in der Matrix enthaltenen Kristalle wird die erwünschte hohe freie Konzentration an Estradiol im Gleichgewicht zum kristallinen Anhydrat aufrechterhalten. Die Nachlieferung von Wirkstoff durch langsame Auflösung der Kristalle sorgt für gleichmäßige Wirkstoffabgabe aufgrund der konstanten thermodynamischen Aktivität.
Soll die direkte Berührung von der Haut mit Wirkstoffpartikeln vermieden werden, wird nur die (hautferne) Matrix mit 17-β-Estradiol-Anhydrat-Kristallen ausgestattet und durch Kaschieren eine hautnahe Klebschicht, die frei von partikulärem Estradiol ist, aufgebracht.

Wird zwischen einer solchen Matrix und der Klebschicht eine für Estradiol schwer durchlässige Membran eingebracht, erreicht man eine stärker vom Pflaster als von der Haut gesteuerte Wirkstoffabgabe.

Da das Einbringen von suspendiertem, mikronisierten Estradiol in den für Acrylsäureestercopolymere üblichen Lösemittelgemischen (Ethanol, Ethylacetat, Methylethylketon etc.) wegen der hohen Löslichkeit hohen Substanzverbrauch bedeutet, kann auch die Verwendung von benzinlöslichen Polymeren, wie Polyisobutylen, besonders vorteilhaft sein.

Kennzeichnend für erfindungsgemäße transdermale therapeutische Systeme ist in jedem Falle die Anwesenheit von kristallinem, also partikulär vorhandenem Estradiol-Anhydrat. Dabei ist der genaue Anteil dieser pseudopolymorphen Erscheinungsform von Estradiol im System unerheblich; entscheidend für die Funktion ist lediglich die Anwesenheit von mindestens ein bis zwei Kristallen pro Quadratmillimeter, damit sich das System im Gleichgewicht mit wasserfrei kristallinem Estradiol befindet, und die Vorteile der erhöhten Wirkstoffabgabe aufweist.

Eine Identifizierung von erfindungsgemäßen TTS ist gegenüber Systemen nach dem Stand der Technik leicht möglich durch polarisationsmikroskopische Feststellung von Estradiol-Kristallen im TTS und die sich gleichzeitig nach Eintauchen in Wasser oder Pufferlösung zumindest zeitweise einstellende Übersättigung des umgebenden Mediums mit Estradiol.

### Beschreibung der Abbildungen:

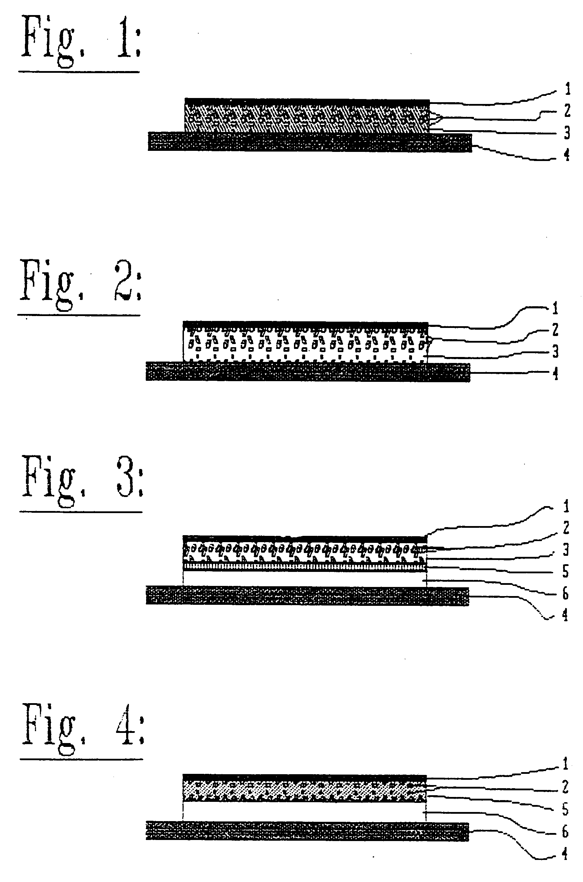

- Fig. 1:: Erfindungsgemäßes System
- 1: - Rückschicht aus Polyester
- 2: - Estradiol-Anhydrat (wasserfreies 17-β-Estradiol)
- 3: - Matrix aus Styrol/Isopren/Styrol-Copolymer und Harz mit gelöstem Wirkstoffanteil
- 4: - Siliconisierte Polyesterfolie als Schutzfolie
- Fig. 2:: Erfindungsgemäßes System
- 1: - Rückschicht aus Polyester
- 2: -Estradiol-Anhydrat (wasserfreies 17-β-Estradiol)
- 3: -Acrylsäureestercopolymer mit gelöstem Wirkstoffanteil
- 4: - Siliconisierte Polyesterfolie als Schutzfolie
- Fig. 3:: Erfindungsgemäßes System
- 1: - Rückschicht aus Polyester
- 2: - Estradiol-Anhydrat (wasserfreies 17-β-Estradiol)
- 3: - Acrylsäureestercopolymer mit gelöstem Wirkstoffanteil
- 4: - Siliconisierte Polyesterfolie als Schutzfolie
- 5: - EVA-Copolymer-Folie
- 6: -Acrylsäureestercopolymer als Klebschicht
- Fig 4:: Erfindungsgemäßes System
- 1: - Rückschicht aus Polyester
- 2: - Estradiol-Anhydrat (wasserfreies 17-β-Estradiol)
- 3: -Acrylsäureestercopolymer mit gelöstem Wirkstoffanteil
- 4: - Siliconisierte Polyesterfolie als Schutzfolie
- 5: - Polyisobutylen mit gelöstem Wirkstoffanteil
- 6: - Acrylsäureestercopolymer als Klebschicht

Die Erfindung wird im folgenden durch Beispiele veranschaulicht:

### Beispiel 1:

Zur Bestimmung der Beeinflussung der Löslichkeit von 17-β-Estradiol durch verschiedene Wasserdampffeuchten wurden zunächst die folgenden wirkstoffreien klebrigen Schichten herangezogen
(Schichtdicke übereinstimmend ca. 80 Mirkometer):
- A.: Acrylatpolymer 1 ohne Zusatz
- B.: Acrylatpolymer 1 mit 12% (g/g) Ölsäure
- C.: Acrylatpolymer 1 mit 5% Ethyllaurat
- D.: Acrylatpolymer 2 ohne Zusatz
- E.: Acrylatpolymer 3 ohne Zusatz
- F.: Acrylatpolymer 4
- G.: Acrylatpolymer 4 mit 10% Isopropylpalmitat
- H.: Acrylatpolymer 5
- I.: Mischung aus 3 Gew.-Teilen Styrol/Isopren-Styrol-Blockcopolymer und 7 Gew.-Teilen eines Kolophomium-Esterharzes mit Erweichungstemperatur 85°C

Zum Test dient eine Testfolie aus Siliconkautschuk enthaltend 8 Gew.-% mikronisiertes 17-β-Estradiol (Dicke 50 Mikrometer).

Legt man Stanzlinge (kreisförmig. 5 cm²) der vorstehenden Kunststofffolien auf je einen 10 cm²-Stanzling des Silicon-Suspensionsträgers luftblasenfrei so auf, daß 17-β-Estradiol aus der Siliconkautschuk/Estradiol-Suspension in die Klebermatrix einwandern kann, so wird der Anteil des 17-β-Estradiols, der nach etwa zwei Wochen Lagerung in die aufgelegte Folie hineindiffundiert, im wesentlichen nur noch von der Sättigungslöslichkeit im Testpolymer bestimmt.
Diese wird folgendermaßen durch die experimentellen Umgebungsbedingungen verändert:
Die Muster werden mit der nötigen Feuchtereguliereinrichtung (s.u.) in siegelfähigen Verbundpackstoff (Papier/Aluminium/Ethylenvinylacetat) eingesiegelt und bei 21°/60% mindestens 10 Tage lang gelagert.

### Feuchteregulatoren:

1. 95% r.F.: eingelegter Streifen Vlies, getränkt mit
   Natriumhydrogenphosphatlösung, gesättigt
2. 65% r.F.: eingelegter Streifen Vlies, getränkt mit
   Natriumnitritlösung, gesättigt
3. 0% r.F.: ca. 10 Körnchen Blaugel. ca. 1g
   Es resultieren also 10 x 3 = 30 Einzelmuster.

Unmittelbar vor der Messung ist der Silicontroger von den Mustern A-I zu entfernen.

### Messung:

Alle 27 Proben werden in 100 ml 0,9%-ige Kochsalzlösung enthaltende Schraubgläser gebracht und nach 5 h Gesamtdauer wird der Versuch unter Probenahme beendet.

Der Gehalt der Muster an Estradiol wird mittels HPLC mit UV-Detektion bestimmt und ergibt aus den Matrix-Stücken eluierte Estradiol-Gehalte von:

| Rel. Feuchte: | 0% | 65% | 95% |
|---|---|---|---|
| | Estradiolgehalt | | |
| A | 0,41% | 0,24% | 0,25% |
| B | 0,38% | 0,23% | 0,19% |
| C | 0,38% | 0,20% | 0,18% |
| D | 0,42% | 0,26% | 0,26% |
| E | 0,39% | 0,30% | 0,34% |
| F | 0,30% | 0,22% | 0,20% |
| G | 0,28% | 0,15% | 0,16% |
| H | 0,54% | 0,27% | 0,32% |
| I | 0,06% | 0,03% | 0,05% |

Die Meßwerte zeigen eine überwiegend deutlich höhere Löslichkeit bei trockener Lagerung. Da die Lagerung relativ kurz dauerte, und kein wasserfreies, sondern Estradiol-semihydrat verwendet wurde, ist davon auszugehen, daß die Löslichkeitssteigerung durch trockene Bedingungen noch weiter gesteigert werden kann.

### Beispiel 2: Herstellung eines erfindungsgemäßen Systems

- 2.0 g: 17-β-Estradiol-semihydrat, mikronisiert
- 60,0 g: Cariflex ^{R} TR 1107 (Styrol-Isopren-Styrol-Blockcopolymer)
- 138,0 g: Foral ^{R} 85 (thermoplastisches Esterharz aus Kolophoniumderivaten
- 200,0 g: Benzin (Siedebereich 80 bis 100 °C)
werden bei Raumtemperatur in einem zylindrischen Glasgefäß bis zum Erreichen einer gleichmäßigen Suspension gerührt und anschließend auf einer kontinuierlich arbeitenden Beschichtungsanlage auf 100 Mikrometer dicke, siliconisierte Polyesterfolie so beschichtet, daß eine Schichtdicke von 100 g/m² (bezogen auf den lösemittelfreien Anteil) resultiert. Der Ausstrich wird jeweils 2,4 min lang bei 40°C, 60 °C, 80 °C und bei 120 °C getrocknet. Sofort wird 15 Mikrometer dicke Polyesterfolie luftblasenfrei unter Walzendruck auf die getrocknete Schicht aufgelegt (zukaschiert).
Durch Stanzung mit Henkellocheisen werden transdermale Systeme von 16 cm² erhalten, die nach Beispiels 5 geprüft wurden.

### Beispiel 3: Herstellung eines Systems noch dem Stand der Technik

- 2,0 g: 17-β-Estradiol-semihydrat, mikronisiert
- 60,0 g: Cariflex ^{R} TR 1107 (Styrol-Isopren-Stryrol-Blockcopolymer)
- 138,0 g: Foral ^{R} 85 (thermoplastisches Esterharz aus Kolophoniumderivaten)
- 200,0 g: Benzin (Siedebereich 80 bis 100°C)
werden bei Raumtemperatur in einem zylindrischen Glasgefäß bis zum Erreichen einer gleichmäßigen Suspension gerührt und anschließend auf einer kontinuierlich arbeitenden Beschichtungsanlage auf 100 Mikrometer dicke, siliconisierte Polyesterfolie so beschichtet, daß eine Schichtdicke von 100 g/m² (bezogen auf den lösemittelfreien Anteil) resultiert. Der Ausstrich wird jeweils 3,6 min lang bei 40°C und bei 60 °C sowie 7,2 min bei 70°C getrocknet. Sofort wird 15 Mikrometer dicke Polyesterfolie luftblasenfrei unter Walzendruck auf die getrocknete Schicht aufgelegt (zukaschiert). Durch Stanzung mit Henkellocheisen werden transdermale Systeme von 16 cm² erhalten, die in Verbundpackstoff aus Papier/Aluminiumfolie/Heißsiegelschicht verpackt und mehr als zwei Wochen später nach Beispiel 5 geprüft wurden.

### Beispiel 4:

### Prüfung der Freisetzung in vitro

Die Freisetzung der transdermalen therapeutischen Systeme wird nach der "paddle-over-disc"-Methode des US-amerikanischen Arzneibuchs (USP) bestimmt. Hierzu wird das zu messende TTS (16 cm²) bei 37°C mit 500 ml Pufferlösung (pH 7,4) überschichtet.

Während der Messung strömt ein Blattrührer mit 150 min ⁻¹ das System konstant an.
Nach 3,8 und 24 Stunden werden Proben der Pufferlösung entnommen und mit HPLC auf ihren Estradiolgehalt (bezogen auf Estradiol-Semihydrat) untersucht.
Es wurden folgende Ergebnisse erhalten:

| TTS Nr. | Freigesetzte Menge Estradiol (mg) | | |
|---|---|---|---|
| | | | |

| Systeme nach Beispiel 2: | | | |
|---|---|---|---|
| | 3h | 8h | 24h |
| 1 | 0,106 | 0,178 | 0,338 |
| 2 | 0,117 | 0,208 | 0,324 |
| 3 | 0,109 | 0,190 | 0,328 |
| 4 | 0,114 | 0,187 | 0,331 |

| Systeme nach Beispiel 3 (Vergleichsbeispiel nach dem Stand der Technik) | | | |
|---|---|---|---|
| | 3h | 8h | 24h |
| 1 | 0,077 | 0,119 | 0,179 |
| 2 | 0,076 | 0,128 | 0,183 |
| 3 | 0,079 | 0,135 | 0,202 |
| 4 | 0,072 | 0,122 | 0,183 |

### Beispiel 5:

### Prüfung der Permeation durch Tierhaut in vitro

In eine auf 37°C thermostatisierte Hautpermeationszelle aus Glas mit 20 ml Akzeptorvolumen und 2,54 cm² freier Diffusionsfläche wird ein ausreichend großes Stück exzidierte haarlose Mäusehaut gespannt, das auf der Unterseite vom Akzeptormedium (Puffer pH 7,4) angeströmt wird und dessen Oberseite (Hornschicht) vorher mit dem zu prüfenden TTS beklebt wurde.
Nach 8 und nach 24 Stunden wird das Medium ausgetauscht und in diesem mit HPLC der Estradiolgehalt bestimmt.
Mehr als 2 Wochen vor der Messung wurde je ein TTS in einen Siegelbeutel enthaltend ca. 1 g vorgetrocknetes Silicagel umgepackt. um den Einfluß von Luftfeuchtigkeit während der Lagerung auszuschließen.
Es ergaben sich folgende Meßwerte (freigesetztes Estradiol in Mikrogramm pro cm²):

| TTS gemäß: | Zeit | | Lagerung |
|---|---|---|---|
| | 8 Stunden | 24 Stunden | |
| Beispiel 2 | 1,7 | 4,3 | ohne |
| Beispiel 3 (Vergleich) | 1,2 | 2,4 | ohne |
| Beispiel 2 | 1,8 | 4,8 | mit |
| Beispiel 3 (Vergleich) | 0,7 | 3,1 | mit Trockenmittel |

Das den Wirkstoff 17-β-Estradiol in kristalliner Form enthaltende TTS unterscheidet sich von solchen den gleichen Wirkstoff in Form eines Semihydrates enthaltenden Präparaten auch dadurch, daß es bei der Lagerung vor Anwendung in einem gasdicht versiegelten Packmittel zusammen mit einem wasserentziehenden bzw. wasseraufnehmenden Mittel vorliegt.

## Patentansprüche

1. Transdermales therapeutisches System enthaltend den Wirkstoff 17-β-Estradiol und gegebenenfalls weitere Wirkstoffe, mit geschichtetem Aufbau auf einer im wesentlichen wirkstoff- und feuchtigkeitsundurchlässigen Rückschicht, einer oder mehrerer Matrixschichten und gegebenenfalls einer Klebschicht, dadurch gekennzeichnet, daß mindestens eine der Matrixschichten oder die Klebschicht wasserfreies 17-β-Estrodiol in kristalliner Form enthält, und daß es bei der Lagerung vor Anwendung in einem gasdicht versiegelten Packmittel zusammen mit einem wasserentziehenden bzw. wasseraufnehmenden Mittel vorliegt.

2. Transdermales therapeutisches System nach Anspruch 1, bestehend aus einer Matrixschicht und einer hautseitigen haftklebenden Klebschicht, dadurch gekennzeichnet, daß die Matrixschicht wasserfreies 17-β-Estradiol in kristalliner Form enthält, die Klebschicht dagegen frei von kristallinem 17-β-Estradiol ist.

3. Transdermales therapeutisches System nach Anspruch 1 oder 2, dadurch gekennzeichnet, daß das Grundmaterial der Klebschicht ein Acrylsäureestercopolymer ist.

4. Transdermales therapeutisches System nach Anspruch 1 oder 2, dadurch gekennzeichnet, daß das Grundmaterial mindestens einer der Matrixschichten Polyisobutylen ist.

5. Transdermales therapeutisches System nach Anspruch 1 oder 2, dadurch gekennzeichnet, daß das Grundmaterial mindestens einer der Matrixschichten ein Acrylsäureestercopolymer ist.

6. Transdermales therapeutisches System nach Anspruch 3 oder 5, dadurch gekennzeichnet, daß das Acrylsäureestercopolymer eine Löslichkeit für den Wirkstoff 17-β-Estradiol (wasserfrei) zwischen 0,4 und 3,0% (g/g) aufweist.

7. Verfahren zur Herstellung eines transdermaler therapeutischen Systems nach einem oder mehreren der Ansprüche 1 bis 6, dadurch gekennzeichnet, daß zuerst eine Suspension von 17-β-Estradiol-Semihydrat in einer Lösung. Dispersion oder Schmelze des Matrix-Grundmaterials bereitet und diese in Form einer Schicht auf ein folienförmiges Basismaterial aufgetragen und die Schicht getrocknet wird, und daß anschließend das so erhaltene Substrat auf 90 bis 175°C erhitzt und dabei eine Umwandlung des 17-β-Estradiol-Semihydrates zu wasserfreiem 17-β-Estradiol in kristalliner Form vorgenommen wird.

8. Verfahren zur Herstellung eines transdermalen therapeutischen Systems nach den Ansprüchen 1 bis 6, dadurch gekennzeichnet, daß zuerst eine Suspension von 17-β-Estradiol-Semihydrat in einer Lösung, Dispersion oder Schmelze des Matrix- bzw. Klebschicht-Grundmaterials bereitet und diese in Form einer Schicht auf ein folienförmiges, vorzugsweise dehäsiv ausgerüstetes Basismaterial aufgetragen und die Schicht gegebenenfalls getrocknet, auf eine im wesentlichen wirkstoff- und feuchtigkeitsundurchlässige Rückschicht aufgelegt und durch Konturstanzen und Folienschnitt vereinzelt und in ein gasdicht versiegeltes Packmittel eingepackt wird, wobei dem Packmittel wasseraufnehmende Mittel hinzugefügt werden, und daß durch einen durch diese über einen längeren Zeitraum vorgenommenen Wasserentzug eine pseudopolymorphe Umwandlung von 17-β-Estradiol-Semihydrat zu 17-β-Estradiol in kristalliner, wasserfreier Form erfolgt.

9. Verfahren zur Herstellung eines transdermalen therapeutischen Systems nach Anspruch 8, dadurch gekennzeichnet, daß die Umwandlung von 17-β-Estradiol-Semihydrat in 17-β-Estradiol in kristalliner, wasserfreier Form innerhalb von höchstens einem Monat Vorlagerung vorgenommen wird.

## Claims

1. Transdermal therapeutic system comprising the active substance 17-β-estradiol and, optionally, further active substances, with a laminated structure positioned on a backing layer which is substantially impermeable to active substances and moisture, one or several matrix layers and, optionally, an adhesive layer, characterized in that at least one of the matrix layers or the adhesive layer contains anhydrous 17-β-estradiol in crystalline form, and that said system, during storage prior to application, is present in a gas-tight, sealed packing material together with a dehydrating or water-absorbing substance.

2. The transdermal therapeutic system according to claim 1, consisting of a matrix layer and a skin-facing, pressure-sensitive adhesive layer, characterized in that the matrix layer contains 17-β-estradiol in crystalline and anhydrous form, whereas the adhesive layer is free from crystalline 17-β-estradiol.

3. The transdermal therapeutic system according to claim 1 or 2, characterized in that the base material of the adhesive layer is an acrylic-acid ester copolymer.

4. The transdermal therapeutic system according to claim 1 or 2, characterized in that the base material of at least one of the matrix layers is polyisobutylene.

5. The transdermal therapeutic system according to claim 1 or 2, characterized in that the base material of at least one of the matrix layers is an acrylic-acid ester copolymer.

6. The transdermal therapeutic system according to claim 3 or 5, characterized in that the acrylic-acid ester copolymer has a solubility for the active substance 17-β-estradiol (anhydrous) of between 0.4 and 3.0% (g/g).

7. Process for the production of a transdermal therapeutic system according to one or more of the claims 1 to 6, characterized in that initially a suspension of 17-β-estradiol semihydrate in a solution, dispersion, or melt of the matrix base material is prepared and applied in the form of a layer on a foliar base substrate material, that the layer is dried, and that subsequently the substrate thus obtained is heated to 90 to 175°C, whereby the 17-β-estradiol semihydrate is converted to anhydrous 17-β-estradiol in crystalline form.

8. The process for the production of a transdermal therapeutic system according to claims 1 to 6, characterized in that initially a suspension of 17-β-estradiol semihydrate in a solution, dispersion or melt of the base material of the matrix or adhesive layer is prepared and is applied in the form of a layer on a foliar, preferably dehesive, base substrate material, that said layer is optionally dried, applied onto a backing layer which is substantially impermeable to active substances and moisture and separated by contour punching and foil cutting and packed in a gas-tight, sealed packing material, whereby water-absorbing substances are added to the packing material, and that, due to the dehydration effected by the water-absorbing substances over a prolonged period of time, a pseudopolymorphic conversion of 17-β-estradiol semihydrate to 17-β-estradiol in crystalline and anhydrous form takes place.

9. The process for the production of a transdermal therapeutic system according to claim 8, characterized in that the conversion of 17-β-estradiol semihydrate to 17-β-estradiol in crystalline and anhydrous form is carried out within a pre-storage period of, at the most, one month.

## Revendications

1. Système thérapeutique par voie transdermique contenant le principe actif 17-β-oestradiol et, le cas échéant, d'autres principes actifs, à structure stratifiée, sur une couche dorsale essentiellement imperméable à la substance active et à l'humidité, d'une ou de plusieurs couches matricielles et, le cas échéant, d'une couche adhésive, caractérisé en ce qu'au moins une des couches matricielles ou la couche adhésive contient du 17-β-oestradiol anhydre sous forme cristalline et en ce qu'il est présent lors de l'entreposage avant l'application dans un agent d'emballage scellé étanche aux gaz conjointement avec un agent d'extraction d'eau, respectivement d'absorption d'eau.

2. Système thérapeutique par voie transdermique selon la revendication 1, constitué par une couche matricielle et par une couche adhésive manifestant une auto-adhésivité du côté tourné vers la peau, caractérisé en ce que la couche matricielle contient du 17-β-oestradiol anhydre sous forme cristalline, tandis que la couche adhésive est exempte du 17-β-oestradiol cristallin.

3. Système thérapeutique par voie transdermique selon la revendication 1 ou 2, caractérisé en ce que la matière de base de la couche adhésive est un copolymère d'ester acrylique.

4. Système thérapeutique par voie transdermique selon la revendication 1 ou 2, caractérisé en ce que la matière de base d'au moins une des couches matricielles est du polyisobutylène.

5. Système thérapeutique par voie transdermique selon la revendication 1 ou 2, caractérisé en ce que la matière de base d'au moins une des couches matricielles est un copolymère d'ester acrylique.

6. Système thérapeutique par voie transdermique selon la revendication 3 ou 5, caractérisé en ce que le copolymère d'ester acrylique présente une solubilité pour le principe actif 17-β-oestradiol (à l'état anhydre) entre 0,4 et 3,0% (g/g).

7. Procédé pour la préparation d'un système thérapeutique par voie transdermique selon une ou plusieurs des revendications 1 à 6, caractérisé en ce qu'on prépare d'abord une suspension du semi-hydrate de 17-β-oestradiol dans une solution, une dispersion ou une masse fondue de la matière de base de la matrice, et on applique cette suspension sous la forme d'une couche sur une matière de base en forme de feuille et on sèche la couche, et en ce qu'on chauffe ensuite le substrat ainsi obtenu à une température de 90 à 175°C tout en procédant en l'occurrence à une conversion du semi-hydrate de 17-β-oestradiol en 17-β-oestradiol anhydre sous forme cristalline.

8. Procédé pour la préparation d'un système thérapeutique par voie transdermique selon une ou plusieurs des revendications 1 à 6, caractérisé en ce qu'on prépare d'abord une suspension du semi-hydrate de 17-β-oestradiol dans une solution, une dispersion ou une masse fondue de la matière de base de la matrice respectivement de la couche adhésive, et on applique cette suspension sous la forme d'une couche sur une matière de base en forme de feuille, de préférence rendue anti-adhésive et on sèche le cas échéant la couche, on la dépose sur une couche dorsale essentiellement imperméable au principe actif et à l'humidité, et on la sépare par estampage du contour et par découpage de la feuille, et on l'emballe dans un agent d'emballage scellé étanche aux gaz, dans lequel on ajoute à l'agent d'emballage un agent absorbant l'eau et en ce que, via une élimination de l'eau à l'aide de ce dernier réalisée pendant un laps de temps prolongé, on obtient une conversion pseudopolymorphe du semi-hydrate de 17-β-oestradiol en 17-β-oestradiol sous forme cristalline anhydre.

9. Procédé pour la préparation d'un système thérapeutique par voie transdermique selon la revendication 8, caractérisé en ce qu'on procède à la conversion du semi-hydrate de 17-β-oestradiol en 17-β-oestradiol sous forme cristalline anhydre au cours d'un préentreposage qui s'étend au maximum sur un mois.
